# EUROPEAN PATENT APPLICATION

(11) **EP 4 005 615 A1**
(43) Date of publication of application: **01.06.2022**
(21) Application number: 19939320.8
(22) Date of filing: 26.07.2019
(51) Int. Cl.: A61M 5/142

(54) **INJECTION PUMP AND INFUSION PUMP**

(71) Applicant: Shenzhen Mindray Scientific Co., Ltd., Shenzhen, Guangdong 518000 (CN)
(72) Inventor: HE, Lijuan, Shenzhen, Guangdong 518000 (CN); CHAI, Haibo, Shenzhen, Guangdong 518000 (CN); CHEN, Dabing, Shenzhen, Guangdong 518000 (CN); DONG, Shenhui, Shenzhen, Guangdong 518000 (CN)
(74) Representative: KIPA AB
(86) International application number: PCT/CN2019/098040
(87) International publication number: WO 2021/016775

(57) **Abstract**

An injection pump (100) and an infusion pump (200). Display areas (1321, 2321) of screen assemblies (132, 232) of the injection pump (100) and the infusion pump (200) extend from the left sides of the center lines of the front sides of pump doors (130, 230) to the right sides of the center lines, and the widths of the display areas (1321, 2321) of the screen assemblies (132, 232) of screen assemblies (132, 232) are greater than the heights thereof; or the widths of the screen assemblies (132, 232) of the screen assemblies (132, 232) are greater than or equal to 70% of the widths of the front sides of the pump doors (130, 230). By using the modes above, the screen assembly (132, 232) may form a roughly elongate display area (1321, 2321). At least part of the display area (1321, 2321) also possesses a touch function, so that some physical buttons (120, 220) used for inputting instructions can be omitted, ensuring that the display area (1321, 2321) is larger, presentation of more information is displayed, and viewing of users is facilitated.

## Description

### TECHNICAL FIELD

The disclosure relates to the technical field of medical device, and more particularly to a structure of an injection and infusion pump.

### BACKGROUND

Generally, in clinical treatment, injection pump and infusion pump are used together, such as be stacked on each other. Some high-end injection and infusion pumps (injection pump and infusion pump) have display screens to display more information. However, due to the increasingly tight space in medical places such as wards and surgical rooms, the compactness requirements for the injection and infusion pump are getting higher and higher. Therefore, the volume of existing injection and infusion pump is generally small. In addition, the existing injection and infusion pump generally adopts the design of small square display screen together with physical keys. Therefore, there is less information displayed on the injection and infusion pump, which is not conducive to viewing more information on the same screen. Moreover, the injection pump should use the syringe to inject. However, the pump door of the existing injection pump blocks an end of the syringe, where the liquid outlet of the syringe locates. During the infusion process, the nurse cannot see the remaining amount of drug in the syringe at the end of the syringe, where the liquid outlet of the syringe locates, resulting in frequent manual opening of the pump door to confirm the remaining amount of drug and to help confirming the time for change or renewal of drug. Moreover, the existing injection pump always has the problems, such as when the syringe and extension pipe fall off and the liquid drug leaks out, the doctor cannot know and find it in time, in clinical treatment.

### SUMMARY

The present disclosure has disclosed an injection pump and an infusion pump which have a larger display area for displaying more information and facilitating a view of a user, without enlarging a shape of the injection pump and the infusion pump.

In a first aspect of an embodiment of the present disclosure, an injection pump is provided, which including:
a pump body, wherein the pump body is provided with an installation structure which is operable to install a syringe;
a drive device, which is operable to move a piston rod of the syringe to inject a liquid;
a control unit, which is operable to control the drive device;
a physical key, which is electrically connected with the control unit, wherein the physical key is operable for a user to input an instruction, and the physical key comprises a power key; and
a pump door, which is movably installed on the pump body and operable to shield and un-shield the syringe, wherein the syringe is installed on the pump body and the pump door includes a door body, a transparent window and a screen assembly, wherein the screen assembly is in communication connection with the control unit and has a display area which is capable of displaying information, wherein at least a part of the display area has a touch-control function and the transparent window is installed on the door body, wherein at least an end of the syringe, where a liquid outlet is placed, is capable of being visually shown through the transparent window, wherein a side of the door body which faces the user is a front surface of the pump door, the screen assembly is arranged on the front surface of the pump door, the display area of the screen assembly extends from a left side of a center line of the front surface of the pump door to a right side of the center line of the front surface of the pump door, wherein a width of the display area of the screen assembly is greater than a height of the display area of the screen assembly.

In a further aspect of an embodiment of the present disclosure, an injection pump is provided, which including:
a pump body, wherein the pump body is provided with an installation structure which is operable to install a syringe;
a drive device, which is operable to move a piston rod of the syringe to inject a liquid;
a control unit, which is operable to control the drive device;
a physical key, which is electrically connected with the control unit, wherein the physical key is operable for a user to input an instruction, and the physical key includes a power key; and
a pump door, which is movably installed on the pump body and operable to shield and un-shield an infusion pipe which is installed on the pump body, wherein the pump door includes a door body, a transparent window and a screen assembly, wherein the screen assembly is in communication connection with the control unit and has a display area which is capable of displaying information, wherein at least a part of the display area has a touch-control function and the transparent window is installed on the door body, wherein at least an end of the syringe, wherein the syringe is installed on the pump body, where a liquid outlet is placed, is capable of visually shown through the transparent window, wherein a side of the door body, which faces the user, is a front surface of the pump door, the screen assembly is arranged on the front surface of the pump door, wherein width of the display area of the screen assembly is greater than or equal to 70% of a width of the front surface of the pump door, and a width of the transparent window is greater than or equal to 30% of the width of the front surface of the pump door.

In a further aspect of an embodiment of the present disclosure, an infusion pump is provided, which including:
a pump body, wherein the pump body is provided with a liquid stopping structure which is operable to stop a liquid which flows through an infusion pipe;
a drive device, which is operable to drive the liquid to flow into the infusion pipe;
a control unit, which is operable to control the drive device;
a physical key, wherein the control unit is electrically connected with the physical key which is operable for a user to input an instruction, and the physical key includes a power key; and
a pump door, which is movably installed on the pump body and operable to shield and un-shield the infusion pipe, wherein the infusion pipe is installed on the pump body, and the pump door comprises a door body and a screen assembly which is capable of displaying information, wherein the screen assembly is in communication connection with the control unit and has a display area which is capable of displaying information, wherein at least a part of the display area has a touch-control function, wherein a side of the door body which faces the user is a front surface of the pump door, the screen assembly is arranged on the front surface of the pump door, the display area of the screen assembly extends from a left side of a center line of the front surface of the pump door to a right side of the center line of the front surface of the pump door, wherein a width of the display area of the screen assembly is greater than a height of the display area of the screen assembly.

In a further aspect of an embodiment of the present disclosure, an infusion pump is provided, which including:
a pump body, wherein the pump body is provided with a liquid stopping structure which is operable to stop a liquid which flows through an infusion pipe;
a drive device, which is operable to drive the liquid to flow into the infusion pipe;
a control unit, which is operable to control the drive device;
a physical key, wherein the control unit is electrically connected with the physical key which is operable for a user to input an instruction, and the physical key includes a power key; and
a pump door, which is movably installed on the pump body and operable to shield and un-shield a syringe which is installed on the pump body, wherein the pump door includes a door body and a screen assembly which is capable of displaying information, wherein the screen assembly is in communication connection with the control unit and has a display area which is capable of displaying information, wherein at least a part of the display area has a touch-control function, wherein a side of the door body which faces the user is a front surface of the pump door, the screen assembly is arranged on the front surface of the pump door, wherein a width of the display area of the screen assembly is greater than or equal to 70% of a width of the front surface of the pump door.

In the injection pump according to some of the above embodiments, the display area of the screen assembly extends from the left side of the center line of the front surface of the pump door to the right side of the center line of the front surface of the pump door and a width of the display area of the screen assembly is greater than a height of the display area of the screen assembly. In the injection pump according to some of the above embodiments, the width of the display area of the screen assembly is greater than or equal to 70% of the width of the front surface of the pump door. In these ways, the display area which has a roughly elongated shape can be formed. Moreover, at least a part of the display area has the touch-control function. In this way, some physical keys used for inputting instructions can be omitted to ensure that the display area is larger and more information can be displayed, so it is more convenient for the user to view. Moreover, the above injection pump has a transparent window. At least the end of the syringe, where a liquid outlet is placed, can be visually shown through the transparent window. Accordingly, the user can easily observe whether there is liquid at the end of the syringe, where a liquid outlet is placed, through the transparent window, so as to determine the injection progress in time and take corresponding measures.

In the infusion pump according to some of the above embodiments, the display area of the screen assembly extends from the left side of the center line of the front surface of the pump door to the right side of the center line of the front surface of the pump door and the width of the display area of the screen assembly is greater than the height of the display area of the screen assembly. In the infusion pump according to some of the above embodiments, the width of the display area of the screen assembly is greater than or equal to 70% of the width of the front surface of the pump door. In these ways, the display area which has a roughly elongated shape can be formed. Moreover, at least a part of the display area has the touch-control function. In this way, some physical keys used for inputting instructions can be omitted to ensure that, the display area is larger and more information can be displayed, so it is more convenient for the user to view.

### BRIEF DESCRIPTION OF THE DRAWINGS

FIG. 1 is a structural diagram for an injection pump (with a closed pump door) in an embodiment of the present disclosure.
FIG. 2 is a structural diagram for an injection pump (with an opened pump door) in an embodiment of the present disclosure.
FIG. 3 is a position diagram showing a proportion of each assembly on a front surface of the pump door of the injection pump in an embodiment of the present disclosure.
FIGS. 4-5 are schematic diagrams for a display interface of a screen assembly in an embodiment of the present disclosure.
FIGS. 6-16 are schematic diagrams showing different shapes and positions of a screen assembly, a physical key and an alarm lamp of the injection pump in an embodiment of the present disclosure.
FIG. 17 is a structural diagram for a screen assembly and a transparent window which are assembled on a frame of a door body in an embodiment of the present disclosure.
FIG. 18 is an exploded diagram for the structure shown in FIG. 17.
FIG. 19 is a sectional diagram for the structure shown in FIG. 17.
FIG. 20 is a partially enlarged diagram of FIG. 19.
FIGS. 21-22 are structural diagrams in which a screen assembly has two and three small screens in an embodiment of the present disclosure.
FIG. 23 is a structural diagram for a front surface of a screen assembly in an embodiment of the present disclosure.
FIG. 24 is a structural diagram for a back surface of a screen assembly in an embodiment of the present disclosure.
FIG. 25 is an exploded diagram for a screen assembly and a frame of a door body in an embodiment of the present disclosure.
FIG. 26 is an exploded diagram for a key cap and a support frame of a physical key in an embodiment of the present disclosure.
FIG. 27 is a sectional diagram for a physical key in an embodiment of the present disclosure.
FIG. 28 is a structural diagram for an infusion pump (with a closed pump door) in an embodiment of the present disclosure.
FIG. 29 is a structural diagram for an infusion pump (with an opened pump door) in an embodiment of the present disclosure.
FIG. 30 is a position diagram showing a proportion of each assembly on a front surface of the pump door of the infusion pump in an embodiment of the present disclosure.
FIGS. 31-45 are schematic diagrams showing different shapes and positions of a screen assembly, a physical key and an alarm lamp of the infusion pump in an embodiment of the present disclosure.

### DESCRIPTION OF THE PREFERRED EMBODIMENTS

The technical solutions in example embodiments of the present disclosure will be described clearly and completely below with reference to the accompanying drawings. In different embodiments, similar elements adopt associated similar reference numbers. In the following embodiments, many details are described in order to make the present disclosure better understood. However, those skilled in the art can easily recognize that some of the features can be omitted in different embodiments, or can be replaced by other elements, materials and methods. In some embodiments, in order to avoid the core part of the present disclosure being inundated by too many descriptions, some operations related to the present disclosure are not shown or described in the specification, as it is not necessary to describe these related operations in detail for those skilled in the art. Because, they can fully understand the relevant operations according to the description in the present disclosure and the general technical knowledge in the field.

In addition, the features, operations or characteristics described in the present disclosure can be combined in any appropriate way to form various embodiments. At the same time, the steps or actions in the method description can also be changed or adjusted in order in a manner obvious to those skilled in the art. Therefore, the various sequences in the present disclosure and the accompanying drawings are only for the purpose of clearly describing an embodiment, and do not mean that they are necessary sequences, unless otherwise specified that one of them must be followed.

The expressions of "first" and "second" recited in this disclosure, are only to distinguish different objects, and do not have any sequence or technical meaning. The terms "connection" and "coupling", as mentioned in this disclosure, include both "direct connection" and "indirect connection (coupling)".

Clinically, the injection pump is usually used in an intravenous infusion environment (such as a vein) that requires maintaining a constant infusion speed and an accurate dosage for a long time. The injection pump is mostly used to inject cardiovascular active drugs, anesthetics, hormone drugs which have little liquid amount but high concentration and should be controlled accurately, such as dopamine, dobutamine, epinephrine, norepinephrine, lidocaine, nitroglycerin, sodium nitroprusside and so on. Of course, the injection pump can also be used to inject other liquid according to the clinical requirement.

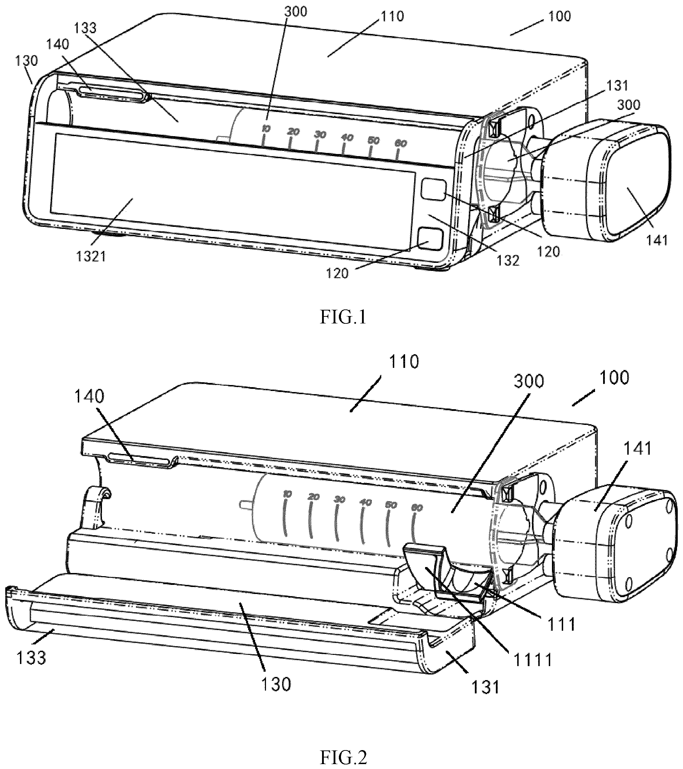

An embodiment of the present disclosure has provided an injection pump. Referring FIG.1 and FIG.2, the injection pump 100 includes a pump body 110, a drive device, a control unit, a physical key 120 and a pump door 130. Wherein the drive device and the control unit are not shown as they are positioned inside the pump body 110 and shielded by other assemblies.

The pump body 110 is provided with an installation structure 1111 which is operable to install a syringe 300. For example, a syringe canister of the syringe 300 is fixed at a certain position by a clamping structure. The installation structure 1111 includes but is not limited to a clamping structure, a magnetic adsorption structure, a snap structure, a tight-fitting structure, etc. The drive device is operable to bring a piston rod of the syringe 300 to move to inject liquid. For example, the piston rod of the syringe 300 is pushed by a push rod 141 shown in the drawing to move. Among them, the drive device can usually use a motor as the power source element. The motor converts a rotation motion into a linear motion through a transmission mechanism (such as a lead screw, nut transmission mechanism), so as to push the piston rod of the syringe 300 to move and squeeze the liquid in the syringe 300. In working principle, the injection action of the injection pump 100 is implemented as follows. The single chip system sends out a control pulse to rotate the motor through a drive circuit. The motor drives the screw rod and nut through a deceleration mechanism to convert the rotation motion of the motor into a linear motion of the nut. The nut is connected with the push rod 141 of the syringe 300 which is matched with the nut itself, that is, the nut can push the piston rod of the syringe 300 which is matched with the nut itself for liquid injection and infusion. By setting a rotation speed of the motor, a push speed of the motor that is applied to the matched syringe 300 can be adjusted, such that an injection dose and speed of the given drug can be adjusted. Of course, in addition to the motor, the drive device can also use a power source element such as a cylinder that can output a linear motion.

The control unit is operable to control the drive device. The control unit may include a processor and a peripheral device interface. The processor can be operable to send an instruction to the drive device. The peripheral device interface can be connected with a peripheral device such as physical key 120 for signal transmission, so that the processor can control the drive device according to the wish of the user.

The control unit is electrically connected with the physical key 120 for the user to input an instruction. Wherein the physical key 120 includes at least one of a power key, an emergency stop key, a main menu key, a screen unlocking key and a pump door unlocking key. The physical key 120 can control the processor through a bus and peripheral device interface. Specifically, the power key is connected with the control unit. By pressing the power key, the control unit is driven to send a control signal to a power system to power on or off the injection pump. Specifically, the emergency stop key is connected with the control unit. By pressing the emergency stop key, the control unit is driven to send a control signal to the drive device to move or stop the drive device of the injection pump. Specifically, the main menu key is connected with the control unit. By pressing the main menu key, the control unit is driven to send a control signal to a display screen control chip for displaying a main injection and infusion interface on a display screen. Specifically, the screen unlocking key is connected with the control unit. By pressing the screen unlocking key, the control unit is driven to send a control signal to a touch-control screen control chip, for activating or deactivating a preset touch-control function or a touch area on a touch-control screen. Specifically, the pump door unlocking key is connected with the control unit. By pressing the pump door unlocking key, the control unit is driven to send a control signal to a pump door switch mechanism for opening or closing the pump door.

The pump door 130 is movably installed on the pump body 110 to shield and un-shield the syringe 300 which is installed on the pump body 110, so as to prevent the syringe 300 from being touched by foreign objects during the operation of the injection pump 100, thus preventing the injection operation from being affected. Referring to FIGS. 1 and 2, the pump door 130 is usually installed on the pump body 110 in a rotatable manner which can be achieved by a hinge connection. In addition, the pump door 130 may also be installed in other movable ways, such as in a slidable or foldable way. When the pump door 130 is opened in a foldable manner, at least one of the transparent window 133 and the screen assembly 132 may also be arranged in a foldable manner if necessary.

Wherein the pump door 130 includes a door body 131, a transparent window 133 and a screen assembly 132. The screen assembly 132 is connected with the control unit (such as the processor), so that it can display specified information under the control of the control unit. At the same time, the screen assembly 132 can also input instructions and information to the control unit through a touch-control manner. The screen assembly 132 has a display area 1321 which is capable of displaying information. Wherein at least a part of the display area 1321 has a touch-control function, that is, at least a part area of the screen assembly 132 is a touch-control display screen which can be connected with the control unit to display information and receive a touch-control instruction of the user. The non-touch-control area of the display area 1321 may only be used for displaying, so it can use an ordinary display screen.

At least a part of the display area 1321 of the screen assembly 132 has a touch-control function. In this way, some physical keys 120 which are used for inputting instructions can be omitted. For example, only important keys, such as the power key 121, emergency stop key 122, etc. can be remained. In this way, the display area 1321 can be larger, so more information can be displayed, so it is more convenient for the user to view.

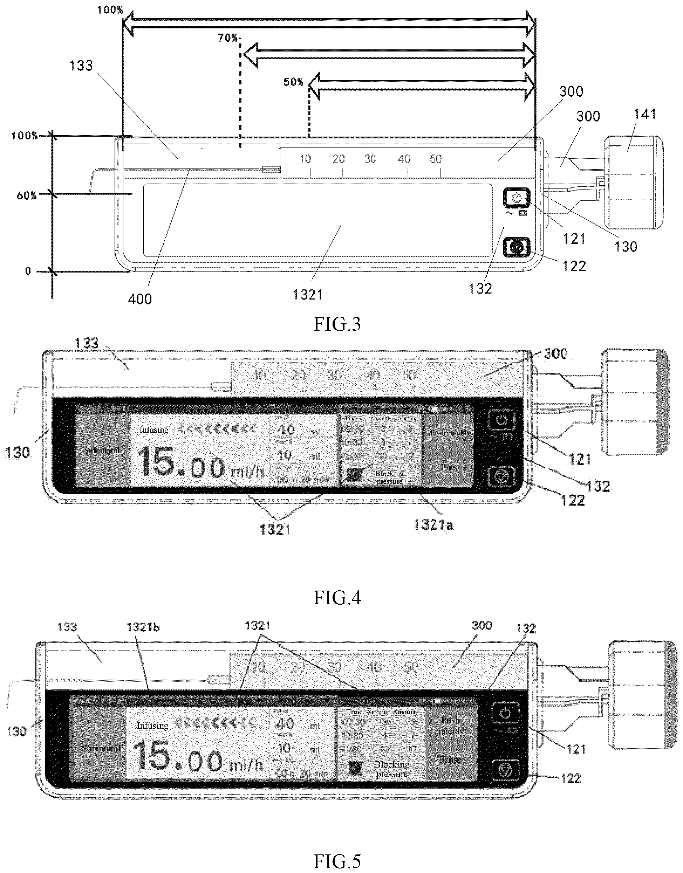

Referring to FIGS. 1 and 2, in one embodiment, a side of the door body 131, which faces the user, is a front surface of the pump door 130, the screen assembly 132 is arranged on the front surface of the pump door 130. The display area 1321 of the screen assembly 132 extends from a left side of a center line of the front surface of the pump door 130 to a right side of the center line of the front surface of the pump door 130, wherein the display area 1321 of the screen assembly 132 has a width which is greater than its height. As shown in FIG. 3, the display area 1321, which extends from the left side of the center line of the front surface of the pump door 130 to the right side of the center line of the front surface of the pump door 130, can be divided into a left display area 1321 and a right display area 1321. The left display area 1321 is positioned on the left side of the center line and the right display area 1321 is positioned on the right side of the center line. The left display area 1321 and the right display area 1321 can be seamlessly connected or arranged at a certain distance from each other.

In this embodiment, the display area 1321 has a roughly elongated shape. Due to the reduction of the original physical key 120 and the full use of the flat shape of the injection pump 100, the surface area of the display area 1321 is greatly enlarged, so that more information can be displayed for the user to view.

In the injection pump 100 according to some of the above embodiments, the display area 1321 of the screen assembly 132 has a width which is greater than or equal to 70% of a width of the front surface of the pump door 130. Since the width of the injection pump 100 is usually greater than its height, the space in the width direction of the injection pump 100 can be sufficiently used to form a display area 1321 which has a roughly elongated shape and such display area 1321 is larger than the display area of the existing product. Thus, the display area 1321 is larger and more information can be displayed, so it is more convenient for the user to view. Herein, the "width" refers to a distance between the left side and the right side of the injection pump 100, in a normal use state. The "height" refers to a distance between the top side and bottom side in the vertical direction of the injection pump 100, in a normal use state.

Of course, in a certain embodiment, the display area 1321 of the screen assembly 132 has a width which is greater than or equal to 70% of a width of the front surface of the pump door 130, and meanwhile the display area 1321 of the screen assembly 132 extends from a left side of a center line of the front surface of the pump door 130 to a right side of the center line of the front surface of the pump door 130. In such a way, the surface area of the display area 1321 of the screen assembly 132 is further enlarged.

Further, please refer to FIGS.1-3, the transparent window 133 is installed on the door body 131, wherein at least an end of the syringe 300, where a liquid outlet of the syringe 300 is placed, can be visually shown through the transparent window 133. Wherein the syringe 300 is installed on the door body 131. Accordingly, the user can easily observe that whether there is liquid at the end of the syringe 300, where the liquid outlet is placed, through the transparent window 133; whether there is a problem in a connection between the end of the syringe 300, where the liquid outlet is placed, and the extension pipe 400 through the transparent window 133. In such a way, the user can determine the injection progress in time and take corresponding measures. The transparent window 133 can be made of transparent materials, such as glass and transparent plastic. In addition, the transparent window 133 can also be directly a hollowed-out window, so that the syringe 300 can be directly displayed in front of the user through the window.

Of course, the injection pump 100 can also include other components, such as a motor drive system, storage system, sensing and monitoring system and alarm system, which will not be described herein.

Referring to FIG. 2, in another embodiment, the installation structure 1111 can include, but is not limited to a clamping mechanism. The installation structure 1111 is positioned inside the pump door 130 to fix the syringe 300.

The clamping mechanism includes a clamping block and an elastic member (such as a spring, shrapnel, etc.) that drives the clamping block to move in a direction for releasing the syringe 300. The clamping block can be movably installed on the pump body 110, on which a syringe placement position is arranged. The syringe 300 is placed on the syringe placement position. The clamping block is positioned inside the pump door 130 and on a movement path of the pump door 130 when the pump door 130 is closed. When the pump door 130 is closed, the pump door 130 pushes the clamping mechanism to clamp the syringe 300 and prevent it from moving. When the pump door 130 is opened, the clamping mechanism is restored to release the syringe 300 as it lost the support of the pump door 130, so that the user can take and place the syringe 300.

As the installation structure 1111 is positioned inside the pump door 130, the outer space of the pump door 130 is released, so that there is more space on the front surface of the pump door 130 to install the screen assembly 132, which is also conducive to arrange the display area 1321 of the screen assembly 132 larger enough.

Further, referring to FIG. 3, in one embodiment, the display area 1321 of the screen assembly 132 has a height which is greater than or equal to 60% of a height of the front surface of the pump door 130. In this way, the proportion of the display area 1321 in the front surface of the pump door 130 can be further enlarged, more information can be displayed. In additional, it is more convenient to implement more personalized settings and displays according to the requirements of the user.

In one embodiment, at least a part of the display area 1321 of the screen assembly has a touch-control function, and information or instructions inputted by the user through the touch-control mode are displayed on the display area 1321 in real time. Referring to FIGS. 4 and 5, the part of the display area 1321 which has the touch-control function can be called as a touch-control area 1321a (which refers to the area shown in the box labeled by the reference number 1321a), while the non-touch-control area in the display area 1321 is called a real-time display area 1321b (which refers to the area shown in the box labeled by the reference number 1321b). The information and instructions inputted by the user in the touch-control area 1321a can be displayed in the display area 1321 in real time. It means that information and instructions inputted by the user in the touch-control area 1321a can be displayed in the entire display area 1321 (including the touch-control area 1321a itself) and can also be displayed only in the real-time display area 1321b. Among them, the real-time display area 1321b and the touch-control area 1321a can be realized by partitioning an entire large screen or can be realized by different small screens respectively.

Preferably, in one embodiment, a surface area of the display area 1321 of the screen assembly 132 is greater than or equal to 2/3 of a surface area of the front surface of the pump door 130. In this way, there can be a larger display area 1321 and touch-control area 1321a (that is, the part of the display area 1321 with the touch-control function).

In another embodiment, the entire display area 1321 of the screen assembly 132 is a touch-control display screen with both information display function and touch-control function. The touch-control display screen can be realized by various screen assemblies that can not only realize the information display function, but also can realize the touch-control function. The entire touch-control screen can be used as the touch-control area 1321a, which can not only display more information, but also enlarge the operation space of the user and greatly improve the convenience of use.

Furthermore, the transparent window 133 is operable to enable the user to easily observe a situation of the pump door 130. Generally, it is mainly for the user to observe the end of the syringe 300, wherein the liquid outlet of the syringe 300 locates. Referring to FIG. 3, in one embodiment, in order to expand the viewing field of the user, in one embodiment, a width of the transparent window 133 is greater than or equal to 30% of the width of the front surface of the pump door 130.

The positions of the transparent window 133 and the screen assembly 132 can be flexibly arranged according to the position of the syringe 300. Please refer to FIG. 3, in one embodiment, the transparent window 133 is positioned at an upper portion of the front surface of the pump door 130, and the screen assembly 132 is positioned below the transparent window 133. At this time, the syringe 300 is placed corresponding to the transparent window 133, such that through the transparent window 133, it is convenient to view the situation in the syringe 300.

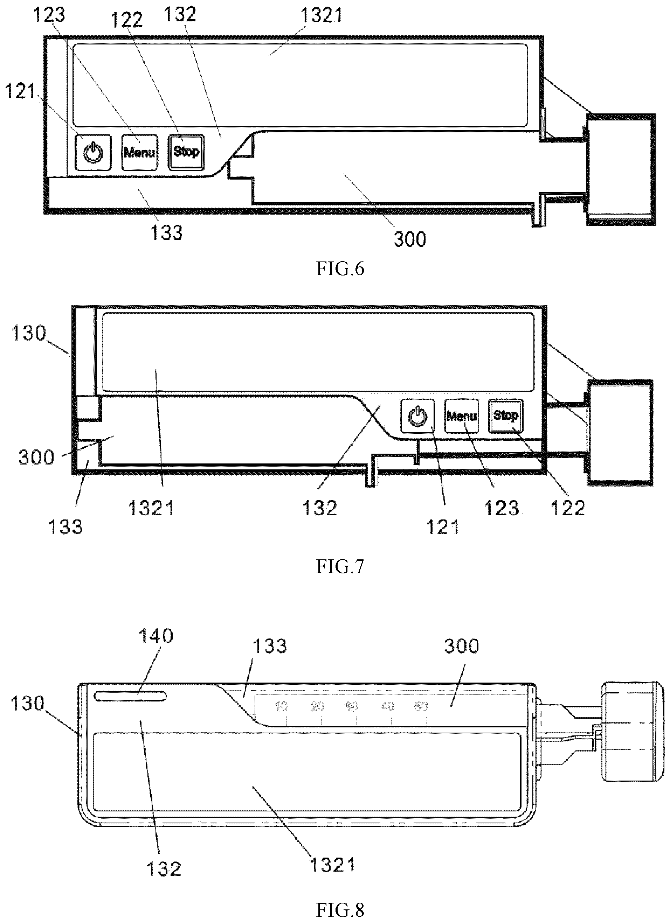

Please refer to FIGS. 6-7, in one embodiment, the transparent window 133 is positioned at a lower portion of the front surface of the pump door 130, and the screen assembly 132 is positioned above the transparent window 133. At this time, the syringe 300 is placed at a lower position inside the pump door 130 corresponding to the transparent window 133.

When the transparent window 133 is positioned below the screen assembly 132, as shown in FIGS. 6 and 7, a left or right portion of the screen assembly 132 extends downward to one side of the transparent window 133 and then is arranged in parallel with the transparent window 133. In FIG. 6, the left portion of the screen assembly 132 protrudes downward. In FIG.7, the right portion of the screen assembly 132 protrudes downward. Similarly, at this time, the downward protruded portion of the screen assembly 132 can directly extend to a frame of the door body 131 or keep a certain distance from the frame of the door body 131 (as shown in FIGS. 6 and 7), so as to ensure that the width of the transparent window 133 can be roughly equal to the width of the front surface of the pump door 130.

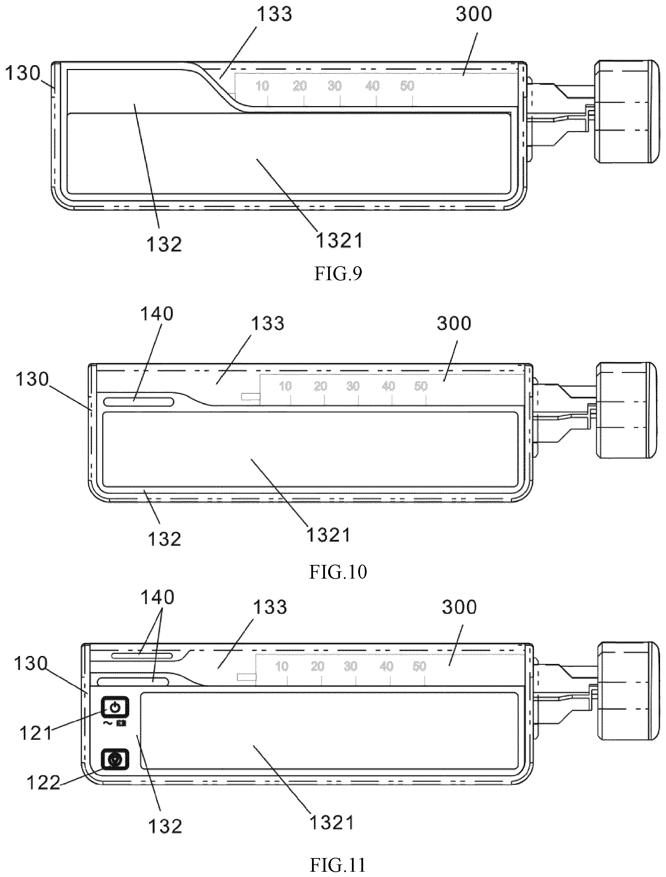

As shown in FIGS. 8-12, when the transparent window 133 is positioned above the screen assembly 132, the left or right portion of the screen assembly 132 extends upward to one side of the transparent window 133 and then is arranged in parallel with the transparent window 133. When the screen assembly 132 protrudes upward, the protruded portion can extend to the frame of the door body 131 as shown in FIGS. 8 and 9 or keep a certain distance from the frame of the door body 131 as shown in FIGS. 10-12. This way can not only enlarge the surface area of the screen assembly 132, but also ensure the size of the transparent window 133 in the width direction, so that the width of the transparent window 133 may be approximately equal to the width of the front surface of the pump door 130, when necessary.

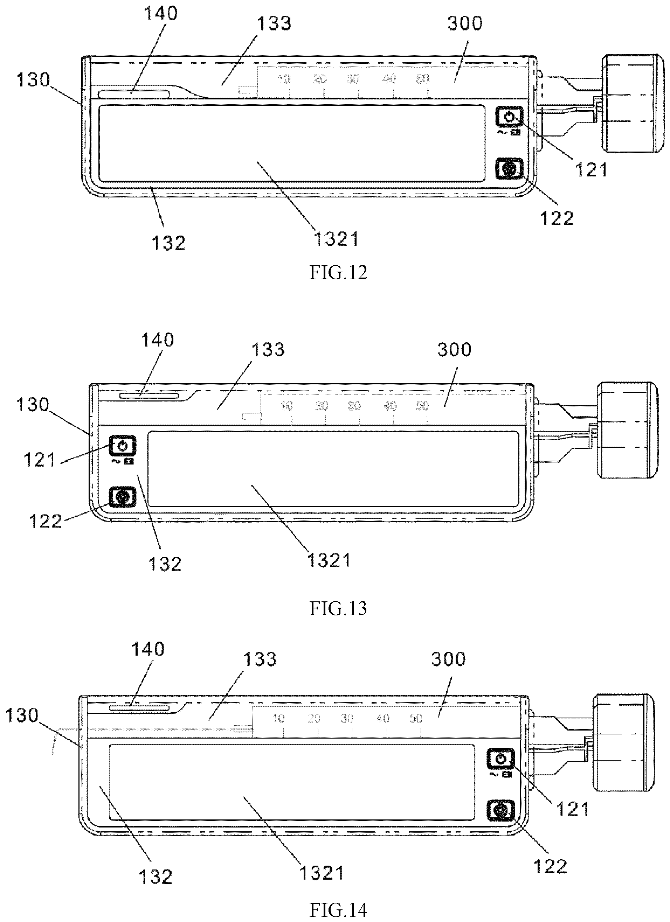

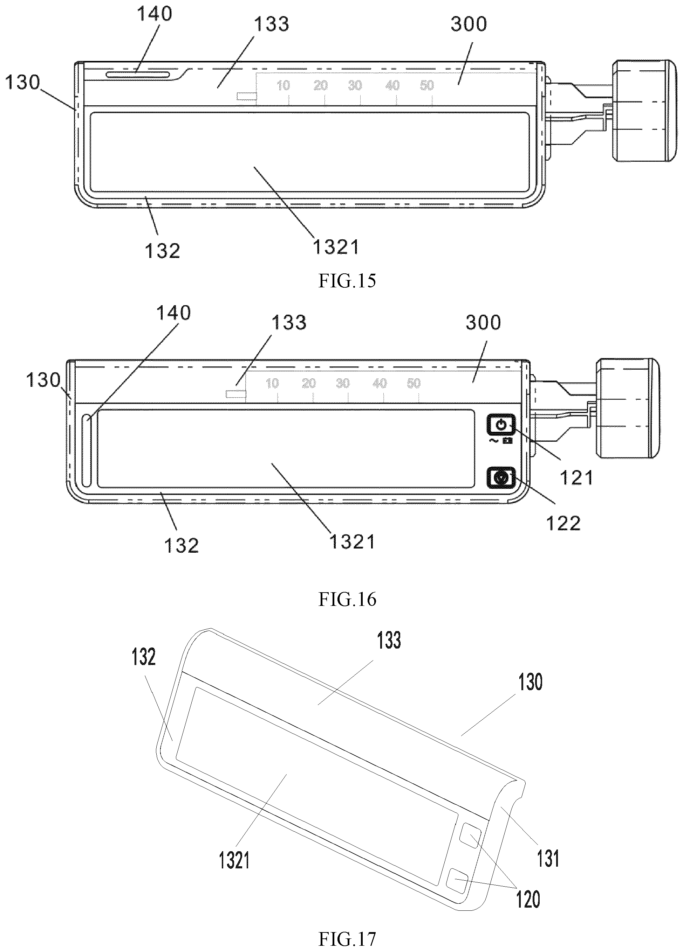

Further, as shown in FIG. 10-16, in some embodiments, an alarm lamp 140 also included. The alarm lamp 140 is arranged above the screen assembly 132, below the screen assembly 132, on a side of the screen assembly 132, or integrated on the screen assembly 132. In FIGS. 8 and 10-12, the alarm lamp 140 is integrated on the protruded portion which is formed by protruding the left portion of the screen assembly 132. In FIGS.13-15, the alarm lamp 140 is arranged at the frame of the door body 131. In FIG. 16, the alarm lamp 140 is integrated on the left side of the screen assembly 132. Of course, the alarm lamp 140 can also be arranged at other positions of the pump door 130 and the pump body 110, which will not be illustrated one by one for example herein.

Further, in order to ensure the sealing performance, please refer to FIG. 17. The transparent window 133 and the screen assembly 132 are jointly sealed with each other to prevent dust and other sundries from entering the pump body 110 from a gap between them. The gap between the transparent window 133 and the screen assembly 132 can be sealed by filling glue, tight fitting, welding and seals.

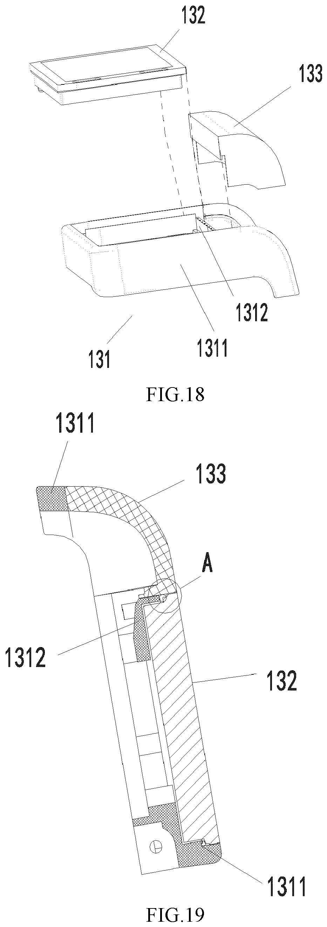

Referring to FIGS. 17 and 18, in one embodiment, the door body 131 has a frame 1311 and a support beam 1312 which is positioned on the frame 1311.

As a scheme with low cost but good sealing effect, in one embodiment, the transparent window 133 is made of transparent plastic, the transparent window 133 and the frame 1311 together form an integrated structure through a two-color injection molding process, and the screen assembly 132 is fixed and sealed with the support beam 1312 and the transparent window 133 by adhesive.

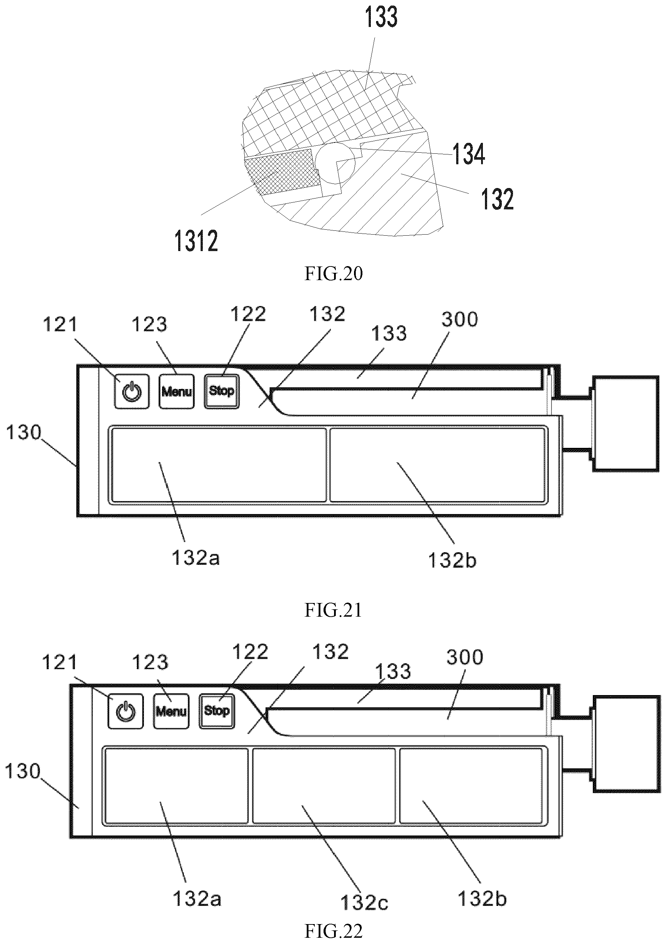

Please refer to FIGS. 18-20, in which FIG.20 is an enlarged view of part A in FIG. 19. The edge of the screen assembly 132 forms a stepped structure, which is sealed and installed on the corresponding frame 1311 and support beam 1312. The gap between the screen assembly 132 and the transparent window 133 is filled with glue 134 to jointly seal the screen assembly 132 and the transparent window 133.

After the screen assembly 132 and the transparent window 133 are jointed, they form an entire structure together with the door body 131. The entire structure can be moved as a whole and can be loaded and unloaded as a whole on the pump body 110. This is very convenient.

The screen assembly 132 may have a large screen (for example, an integral touch-control display). In addition, please refer to FIGS. 21 and 22. In one embodiment, the screen assembly 132 may also have at least two independent small screens. Two adjacent small screens are separated, or two adjacent small screens are separated by an opaque frame. In FIG. 21, the screen assembly 132 has two independent small screens 132a and 132b. In FIG. 22, the screen assembly 132 has three independent small screens 132a, 132b and 132c.

In some embodiments, at least one of these small screens can be selected as an ordinary display screen for displaying information only, and at least one of these small screens can be selected as a touch-control display screen that can display information and realize a touch-control function. Under the combination of these small screens, both display and touch-control functions can be completed. For example, some small screens can be used as the aforementioned real-time display area 1321b, and some small screens can be used as the aforementioned touch-control area 1321a.

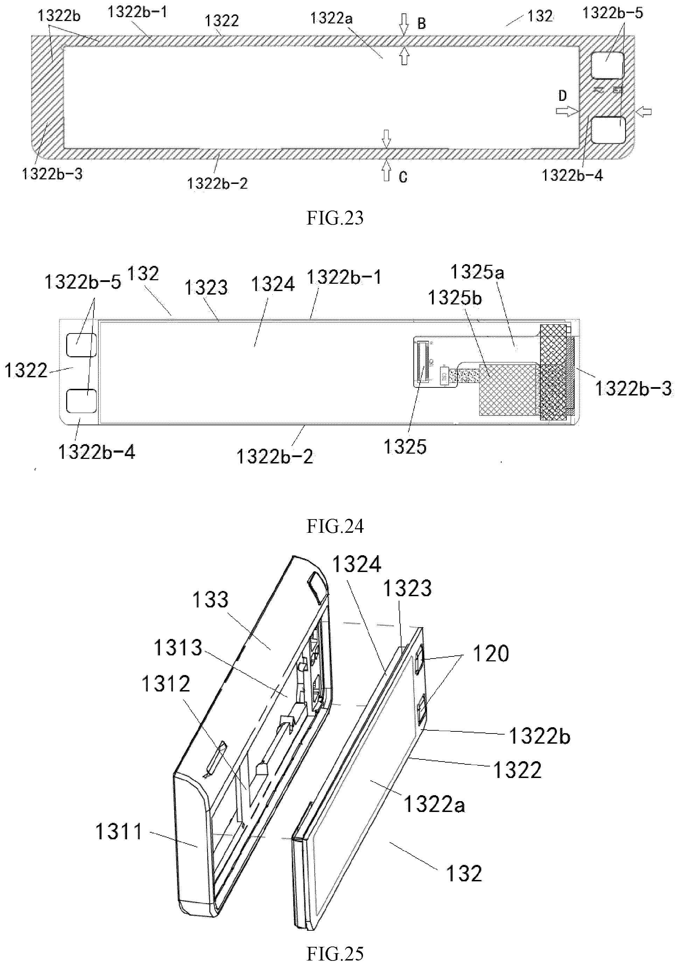

Further, referring to FIGS. 23-26, in one embodiment, the screen assembly 132 includes an outer panel 1322, a touch-control screen 1323 and a display screen 1324. The outer panel 1322 has a transparent area 1322a and an opaque area 1322b which is arranged around the transparent area 1322a. The outer panel 1322 may be made of transparent glass, plastic or other materials. The display area of the display 1324 is exposed from the transparent area 1322a to display relevant information. The opaque area 1322b has an upper area 1322b-1, a lower area 1322b-2, a first side area 1322b-3 and a second side area 1322b-4. The first side area 1322b-3 and the second side area 1322b-4 are positioned between the upper area 1322b-1 and the lower area 1322b-2. The opaque area 1322b is generally in a rectangle shape. Of course, it can also be in other shapes, such as a circular or special shape. Generally, the display screen 1324 is correspondingly provided with a display control chip (which is blocked in the drawings and not shown), and the touch-control screen 1323 is correspondingly provided with a touch-control chip (which is blocked in the drawings and not shown). Among them, in the common display screen, there are multiple display control chips which are operable to send or receive signals respectively. Due to the large number of the display control chips and the touch-control chips, it is difficult to find a place on the pump door to put down all these chips. Therefore, in the prior injection pumps, the display control chip and the touch-control chip are assembled on the upper or lower side of the back surface of the door body 131. In order not to expose these control chips, the opaque area 1322b should shield the control chips, which results in that the size of the upper area 1322b-1 and the lower area 1322b-2 of the opaque area 1322b should be wide. Generally, the width of the upper area 1322b-1 or lower area 1322b-2 is difficult to be less than 40mm. In addition, the height of the injection pump 100 itself is not high, so that the display area 1321 of the screen assembly 132 is further compressed in the height direction, which is not conducive to the information display.

In this regard, in this embodiment, a send terminal and a receive terminal of the display screen 1324 are integrated on one display control chip, which reduces the number of the display control chips and reduces the assembly space required for the display control chips and the touch-control chips. Furthermore, referring to FIG. 24, the display control chip and the touch-control chip are arranged on a back surface of the first side area 1322b-3. The first side area 1322b-3 can be the left side or the right side of the outer panel 1322.

This structure can release the upper area 1322b-1, the lower area 1322b-2 and the second side area 1322b-4 of the opaque area 1322b in the outer panel 1322. And the width of at least one of the upper area 1322b-1, the lower area 1322b-2 and the second side area 1322b-4 of the opaque area 1322b can be reduced (the size as shown in the directions B, C and D in FIG. 3) according to the requirement. In such a way, the display area 1321 of the outer panel 1322 can be made larger without changing the size of the outer panel 1322. Thus, the corresponding display screen 1324 can also be made larger such that the surface area of the display area 1321 can be enlarged.

In this way, the display area 1321 of the screen assembly 132 can be as close to an outer edge of the opaque area 1322b on the upper and lower sides as possible, so as to realize the effect of narrow frame. This structure makes full use of the width of the pump door 130 and further improves the height of the display area 1321 of the screen assembly 132 without changing the volume of the pump door 130. Based on this structure, in one embodiment, the width of the area, where the control chip is not arranged, in the opaque area 1322b, such as the width of the upper area 1322b-1, the lower area 1322b-2 and the second side area 1322b-4, can be arranged to 2-4mm at least, such as 3.3mm.

Furthermore, in one embodiment, a screen test interface is arranged on the back surface of the first side area 1322b-3. Wherein the screen test interface is connected with the display control chip and the touch-control chip. Among them, the display control chip, touch-control chip and screen test interface are arranged on the same side, which is convenient for outgoing line. The space of the screen test interface on single side can be less than 12mm, preferably 6mm, so as to reduce the space that is occupied by the screen test interface. At the same time, the space occupied by the non-display area 1321 above and below the opaque area 1322b is the smallest, so that the upper and lower sides of the opaque area 1322b are extremely narrow, such as less than 40mm.

This embodiment uses a monolithic integrated chip, which is placed on the side and integrates the drive terminal and the receive terminal. Moreover, in this embodiment, the test interface is arranged on one side, and the width of the test interface is arranged to be less than 12mm. Based on this structure, when using the scheme shown in the above embodiment, the display 1324 can generally adopt the COG process with lower cost, that is, the chip on glass process, which can reduce the cost and satisfy the requirements of narrow frame and overall coordination.

Please continue to refer to FIG. 24. In one embodiment, in order to further reduce the space of the control chip, the interface of the display 1324 and the interface of the touch-control screen 1323 are integrated into an integrated interface 1325.

The display control chip has one interface cable 1325a, and the touch-control chip has one interface cable 1325b. The two interface cables are integrated into an integrated interface 1325 which integrates display and touch functions, so there is no need to arrange their own interfaces separately.

In order not to occupy more space of the first side area 1322b-3, please refer to FIG. 24. In one embodiment, the integrated interface 1325 is arranged from the first side area 1322b-3 to the center of the display screen 1324 along a length direction of the display screen 1324. In this way, the integrated interface 1325 can be positioned on the back surface of the display 1324 without exceeding the periphery of the display 1324, so as to avoid increasing the width of other sides of the opaque area 1322b.

In one embodiment, the size of the display area 1321 is 7 inches, so that more information can be displayed for the user to view.

In one embodiment, when the display area 1321 is enlarged, in order to ensure the display quality, in one embodiment, the screen resolution of the display 1324 is not less than 960^{∗}272.

In order to make full use of the feature that the length of the pump door is larger than its width, in one embodiment, the length of the display area 1321 is larger than its width for more than 5 times, for example, its length-width ratio is 6:1.

Referring to FIGS. 6-7, 11-14 and 16, in some embodiments, the physical key 120 is arranged on the front surface of the pump door 130 and is positioned above the screen assembly 132, below the screen assembly 132, on the side of the screen assembly 132 or integrated on the screen assembly 132. The physical key 120 integrated in the screen assembly 132 may also be arranged at an upper portion, a lower portion or a side of the screen assembly 132.

Please continue to refer to FIGS. 1 and 3. In this embodiment, some or all physical keys 120 are also arranged on the outer panel 1322 at the side which is opposite to the control chip 1324 (the position of the keyhole 1322b-5 shown in the drawing, that is the second side area). In this layout, the control chip 1324 and the physical key 120 (the position of the keyhole 1322b-5 shown in the drawing) are respectively arranged on both sides of the screen 1323. This layout is relatively fixed and more in line with the requirements of man-machine operation. It can realize the narrow side frame design of the upper area 1322b-1 and the lower area 1322b-2 of the screen assembly 132 and enlarge the display area 1321, while making the entire screen assembly 132 to be coordinated with the physical key 120 and in line with the ergonomic habit of the user.

As the touch control screen itself is made of glass, it is complicated to add physical keys 120 to the screen. Therefore, generally, the keys of the touch-control display screen use the touch function, and sound, light and vibration are added to improve the perception for the keys of the customer. However, this way cannot achieve the effect of the physical key 120. At the same time, the reliability is not as good as the physical key 120, and the recognizability is not as strong as the physical key 120. However, the physical keys 120 of some non-touch-control display screen are difficult to prevent the liquid or stain residues on the keys. Although the problem of liquid residue can be solved by paster, it is still difficult to achieve the transparent effect of glass in terms of appearance and meanwhile achieve the effect of touch-control display screen.

Further, referring to FIGS. 5-7, the physical key 120 includes a key switch 126 connected with the control unit, a key cap 124 sheathed on the key switch 126, and a support frame 125 which supports the key cap 124 from inside. The key switch 126 is positioned inside the outer panel 1322. A portion of the key cap 124 extends from an inner side of the outer panel 1322 and protrudes from the keyhole 1322b-5 for the user to press. The key cap and the key switch can be movably contacted or disconnected according to the pressure on the key cap. The support frame 125 fixes the key cap 124 in the keyhole 1322b-5. The key cap 124 is matched and sealed with an opening wall of the keyhole 1322b-5 to achieve the effect of waterproof and dustproof.

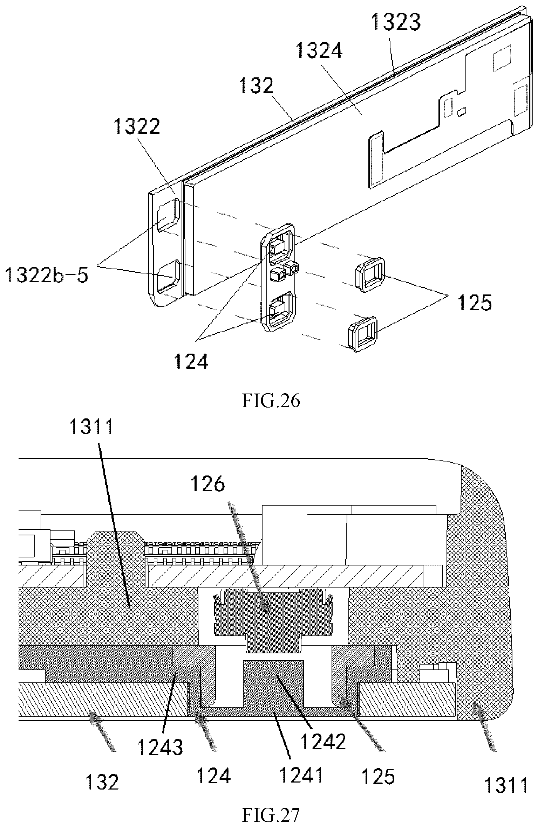

In one embodiment, referring to FIG. 27, one end of the support frame 125 abuts against the frame 1311 of the door body 131, and the other end of the support frame 125 abuts against the key cap 124, so that the key cap 124 is suspended. The arrangement of the support frame 125 is convenient for assembly and processing. In additional, this arrangement can easily fix the key cap 124 on the outer panel 1322 of the screen assembly 132.

Of course, in some embodiments, the support frame 125 can also be omitted, and the key cap 124 can be directly fixed on and connected with the outer panel 1322 or the door body 131 (such as the frame 1311 or the support beam 1312) by other means, such as welding, clamping or other fixing methods, so that it is fixed inside the opening wall of the keyhole 1322b-5.

Further, please refer to FIGS. 25-27. The key cap 124 has a pressing portion 1241 and an installation portion 1243 which is bent from an outer circumference of the pressing portion 1241. The installation portion 1243 is positioned between the door body 131 and the screen assembly 132 and can be clamped by the door body 131 (specifically the center of the frame 1311) and the screen assembly 132 (specifically the outer panel 1322), so as to prevent the key cap 124 from shifting. Of course, the key cap 124 may also be released between the door body 131 (specifically, the frame 1311 or the support beam 1312) and the screen assembly 132 (specifically, the outer panel 1322). The surface of the pressing portion 1241, which faces the key switch 126, has a protruded abutting block 1242. Under normal circumstances, the abutting block 1242 can be separated from the key switch 126 or without force transmission with the key switch 126. When the key cap 124 is pressed, the abutting block 1242 can press the key switch 126 and trigger the key switch 126.

The key cap 124 can be made of elastic material, preferably flexible material with elasticity, so as to improve the pressing feel. In one embodiment, the support frame 125 supports the inner wall of the key cap 124 tightly so that the key cap 124 is tightly matched with the opening wall of the keyhole 1322b-5 to realize the sealing connection. Of course, in some embodiments, the sealing connection between the key cap 124 and the outer panel 1322 can also be realized by arranging a seal and/or filling glue.

This design can integrate the physical key 120 with the screen assembly 132 or an outer cover. In particular, the physical key 120 can be integrated on the screen assembly 132 without damaging the screen. On the basis of ensuring that the screen effect is not affected, the hand feel of the physical key 120 can be satisfied while preventing the liquid inflow and facilitating cleaning. Meanwhile, the requirements of frequent disinfection and cleaning of medical devices can be satisfied. In addition, since multiple infusion pumps and/or injection pumps are often used in combination with each other in the prior art, multiple infusion pumps and/or injection pumps are often stacked one above another or even stacked one above another in an installation frame, due to space constraints. In this case, the infusion pump and/or injection pump will usually only expose their front surfaces. In this scheme, the physical key 120 is integrated with the screen assembly 132 and arranged on the front surface of the infusion pump or injection pump, which can facilitate the pressing operation of the user. Especially in case of medical emergency, the medical staff can easily operate the keys on the front surface of the infusion pump or injection pump.

In one embodiment, the shortest distance from the transparent area 1322a of the outer panel 1322 to the hole wall of the keyhole 1322b-5 is 2-4mm. The surface area of the transparent area 1322a can be enlarged as much as possible without changing the size of the outer panel 1322, so as to expose more display areas of the display screen 1324.

In one embodiment, an outer surface of the pressing portion 1241 of the key cap 124 is level with an outer surface of the outer panel 1322, so that the outer surface of the screen assembly 132 maintains a good flatness. In such a way, the screen assembly 132 and the physical key 120 form an integral plane, which is simple and natural, and easy to clean.

Of course, the injection pump 100 shown in FIGS. 1 and 2 is only an example. In other embodiments, the screen assembly 132 and the physical key 120 may also be arranged on the pump body 110. Alternatively, in some embodiments, the injection pump 100 may not have a pump door 130. The entire injection pump 100 is an overall structure similar to the shape of the pump body 110, and the screen assembly 132 and the physical key 120 may also be arranged on the overall structure at this time.

Based on the above technical concept, an embodiment of the present disclosure also provides an infusion pump. Different from the above injection pump 100, the infusion pump is operable to drive an infusion pipe for infusing liquid according to a preset time and speed.

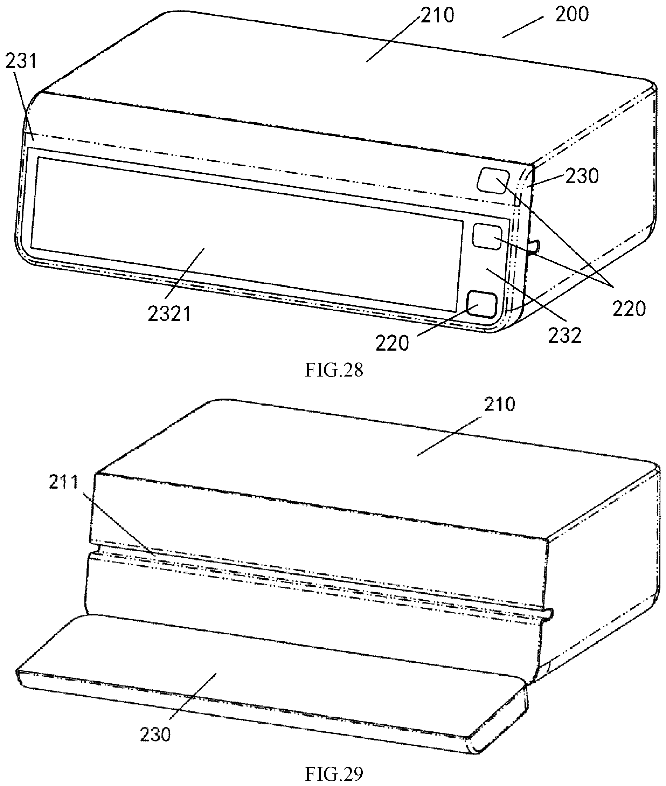

In one embodiment, referring to FIGS. 28 and 29, the infusion pump includes a pump body 210, a drive device, a control unit, a physical key 220 and a pump door 230. Wherein the drive device and the control unit are not shown.

The pump body 210 is provided with a placement position 211 for placing an infusion pipe, as well as a liquid stopping structure (which is unshown in the drawing) which is operable to place and cut off the infusion pipe during a non-working phase for preventing the liquid which flows through the infusion pipe to enter into the body of the patient excessively. Under the normal infusion state, the liquid in the infusion pipe can flow freely. When the pump door 230 is opened, the liquid stopping structure clamps the infusion pipe to stop the flow of liquid in the infusion pipe. The liquid stopping structure is linked with the pump door 230, and the cut-off state of the liquid stopping structure to the infusion pipe is controlled by the movement of the pump door 230. For example, when the pump door 230 is opened, the liquid stopping structure is driven to cut off the infusion pipe. When the pump door 230 is closed, the liquid stopping structure can release the infusion pipe or still be in the state of cutting off the infusion pipe, and the control unit controls the time when the liquid stopping structure cuts off the infusion pipe. The liquid stopping structure includes but is not limited to a clamping structure of a liquid stopping clamp. The drive device is operable to drive the liquid to flow into the infusion pipe. Among them, the drive device can usually adopt a peristaltic pump or other device that can drive the movement of liquid in the infusion pipe. The peristaltic pump can include a motor and a corresponding peristaltic mechanism.

The control unit is operable to control the drive device. The control unit may include a processor and a peripheral device interface. The processor can be operable to send an instruction to the drive device. The peripheral device interface can be connected with a peripheral device such as physical key 220 for signal transmission, so that the processor can control the drive device according to the wish of the user.

The control unit is electrically connected with the physical key 220 for the user to input an instruction. Wherein the physical key 220 includes at least one of a power key, an emergency stop key, a main menu key, a screen unlocking key and a pump door unlocking key. The physical key 220 can control the processor through the bus and peripheral device interface. Specifically, the power key is connected with the control unit. By pressing the power key, the control unit is driven to send a control signal to a power system to power on or off the infusion pump. Specifically, the emergency stop key is connected with the control unit. By pressing the emergency stop key, the control unit is driven to send a control signal to the drive device to move or stop the drive device of the infusion pump. Specifically, the main menu key is connected with the control unit. By pressing the main menu key, the control unit is driven to send a control signal to a display screen control chip for displaying a main injection and infusion interface on a display screen. Specifically, the screen unlocking key is connected with the control unit. By pressing the screen unlocking key, the control unit is driven to send a control signal to a touch-control screen control chip, for activating or deactivating a preset touch-control function or a touch area on a touch-control screen. Specifically, the pump door unlocking key is connected with the control unit. By pressing the pump door unlocking key, the control unit is driven to send a control signal to a pump door switch mechanism for opening or closing the pump door. Generally, the user configure the drugs, the flow speed and capacity of the liquid required by the patient according to the advice of the doctor through the human-machine interface of the infusion pump and selects the infusion apparatus, that can be used together, according to the brand and specification of the infusion apparatus, which is indicated by the infusion pump. The control system of the infusion pump automatically converts the preset characteristic parameters and flow data of the infusion apparatus into the operation parameters of the drive motor. The drive motor drives a camshaft of the peristaltic mechanism to rotate through a deceleration mechanism. The rotation of the camshaft drives a group of pump vanes to implement a linear reciprocation motion. The group of pump vanes cooperate with an extrusion plate to squeeze and release the outer wall of the infusion apparatus in sequence for driving the liquid in the infusion pipe to flow directionally and continuously. In such a way, the quantitative liquid infusion with a constant speed can be obtained.

The pump door 230 is movably installed on the pump body 210 to shield and un-shield the infusion pipe which is installed on the pump body 210, so as to prevent the infusion pipe from being touched by foreign objects during the operation of the infusion pump, thus preventing the infusion operation from being affected. Referring to FIGS. 28 and 29, the pump door 230 is usually installed on the pump body 210 in a rotatable manner which can be achieved by a hinge connection. In addition, the pump door 230 may also be installed in other movable ways, such as in a slidable or foldable way. When the pump door 230 is opened in a foldable manner, the screen assembly 232 may also be arranged in a foldable manner if necessary.

Wherein the pump door 230 includes a door body 231 and a screen assembly 232, wherein the screen assembly 232 is connected with the control unit (such as the processor), so that it can display specified information under the control of the control unit. At the same time, the screen assembly 232 can also input instructions and information to the control unit through a touch-control manner. The screen assembly 232 has a display area 2321 which is capable of displaying information. Wherein at least a part of the display area 2321 has a touch-control function, that is, at least part area of the screen assembly 232 is a touch-control display screen which can be connected with the control unit to display information and receive a touch-control instruction of the user. The non-touch-control area of the display area 2321 may only be used for displaying, so it can use an ordinary display screen.

At least a part of the display area 2321 of the screen assembly 232 has a touch-control function. In this way, some physical keys 220 used for inputting instructions can be omitted. For example, only important keys, such as the power key 221, emergency stop key 222, etc., can be remained. In this way, the display area 2321 can be larger, so more information can be displayed, so it is more convenient for the user to view.

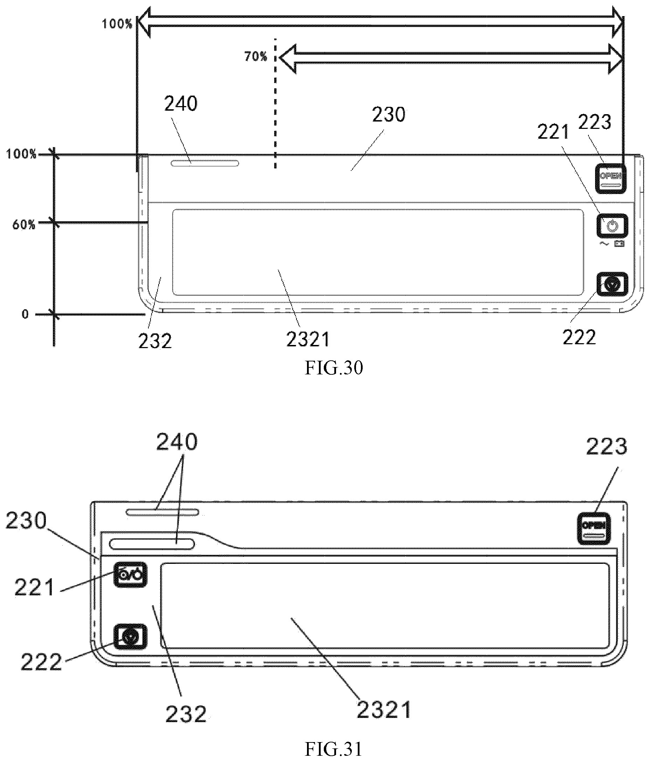

Referring to FIGS. 28-30, in one embodiment, a side of the door body 231 which faces the user is a front surface of the pump door 230, the screen assembly 232 is arranged on the front surface of the pump door 230. The display area 2321 of the screen assembly 232 extends from a left side of a center line of the front surface of the pump door 230 to a right side of the center line of the front surface of the pump door 230, wherein the display area 2321 of the screen assembly 232 has a width which is greater than its height. As shown in FIG. 30, the display area 2321 which extends from the left side of the center line of the front surface of the pump door 230 to the right side of the center line of the front surface of the pump door 230, can be divided into a left display area and a right display area. The left display area is positioned on the left side of the center line and the right display area is positioned on the right side of the center line. The left display area and the right display area can be seamlessly connected or arranged at a certain distance.

In this embodiment, the display area 2321 has a roughly elongated shape. Due to the reduction of the original physical key 220 and the full use of the flat shape of the infusion pump, the surface area of the display area 2321 is greatly enlarged, so that more information can be displayed for the user to view.

In the infusion pump according to some of the above embodiments, the display area 2321 of the screen assembly 232 has a width which is greater than or equal to 70% of a width of the front surface of the pump door 230. Since the width of the infusion pump is usually greater than its height, the space in the width direction of the infusion pump can be sufficiently used to form a display area 2321 which has a roughly elongated shape and such display area 2321 is larger than the display area of the existing product. Thus, the display area 2321 is larger and more information can be displayed, so it is more convenient for the user to view. Herein, the "width" refers to a distance between the left side and right side of the infusion pump, in a normal use state. The "height" refers to a distance between the top side and bottom side in the vertical direction of the infusion pump, in a normal use state.

Of course, in a certain embodiment, the display area 2321 of the screen assembly 232 has a width which is greater than or equal to 70% of a width of the front surface of the pump door 230, and meanwhile the display area 2321 of the screen assembly 232 extends from a left side of a center line of the front surface of the pump door 230 to a right side of the center line of the front surface of the pump door 230. In such a way, the surface area of the display area 2321 of the screen assembly 232 is further enlarged.

Of course, the infusion pump can also include other components, such as a plunger pump drive system, storage system, sensing and monitoring system and alarm system, which will not be repeated herein.

Furthermore, referring to FIG. 30, in one embodiment, the display area 2321 of the screen assembly 232 has a height which is greater than or equal to 60% of a height of the front surface of the pump door 130. In this way, the proportion of the display area 2321 in the front surface of the pump door 130 can be further enlarged, and more information can be displayed. In additional, it is more convenient to implement more personalized settings and displays according to the requirements of the user.

In one embodiment, at least a part of the display area 2321 of the screen assembly has a touch-control function, and information or instructions inputted by the user through the touch-control mode are displayed on the display area 2321 in real time. Referring to FIGS. 4 and 5 (although FIGS. 4 and 5 show the injection pump, however their distribution of the display area 1321 can also be applied to the infusion pump, and the structure of the display area 1321 can be the structure of the display area 2321 in this embodiment), the part of the display area 1321 which has the touch-control function can be called as a touch-control area 1321a, while the non-touch-control area in the display area 1321 can be called a real-time display area 1321b. The information and instructions inputted by the user in the touch-control area 1321a can be displayed in the display area 1321 in real time. It means that information and instructions inputted by the user in the touch-control area 1321a can be displayed in the entire display area 1321 (including the touch-control area 1321a itself) and can also be displayed only in the real-time display area 1321b. Among them, the real-time display area 1321b and the touch-control area 1321a can be realized by partitioning an entire large screen or can be realized by different small screens respectively.

Preferably, in one embodiment, the surface area of the display area 2321 of the screen assembly 232 is greater than or equal to 2/3 of the surface area of the front surface of the pump door 130. In this way, there can be a larger display area 2321 and touch-control area 1321a (that is, the part of the display area 2321 with a touch-control function).

In another embodiment, the entire display area 2321 of the screen assembly 232 is a touch-control display screen with both information display function and touch-control function. The touch-control display screen can be realized by various screen assemblies that can not only realize the information display function, but also realize the touch-control function. The entire touch-control screen can be used as the touch-control area 1321a, which can not only display more information, but also enlarge the operation space of the user and greatly improve the convenience of use.

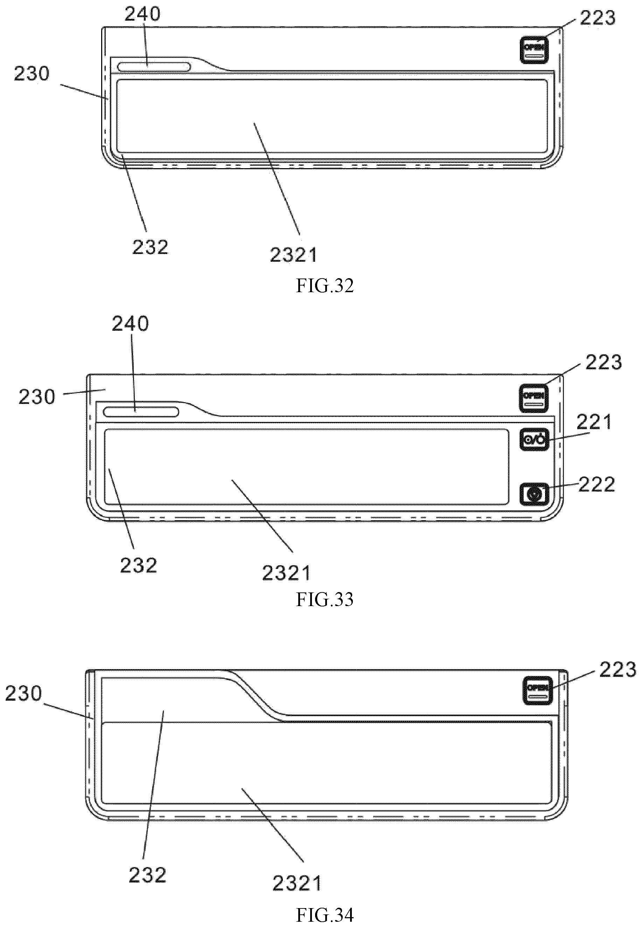

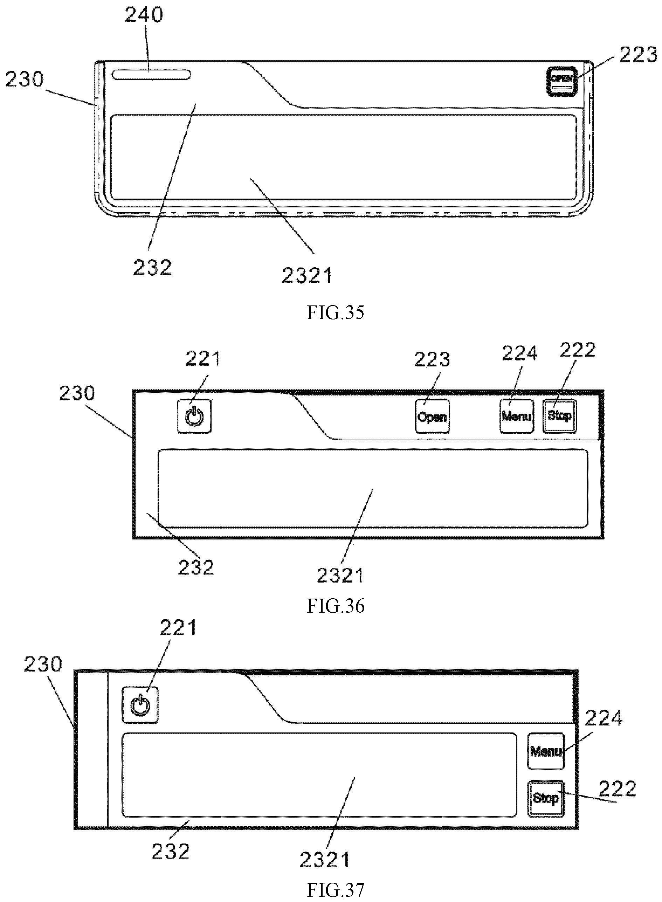

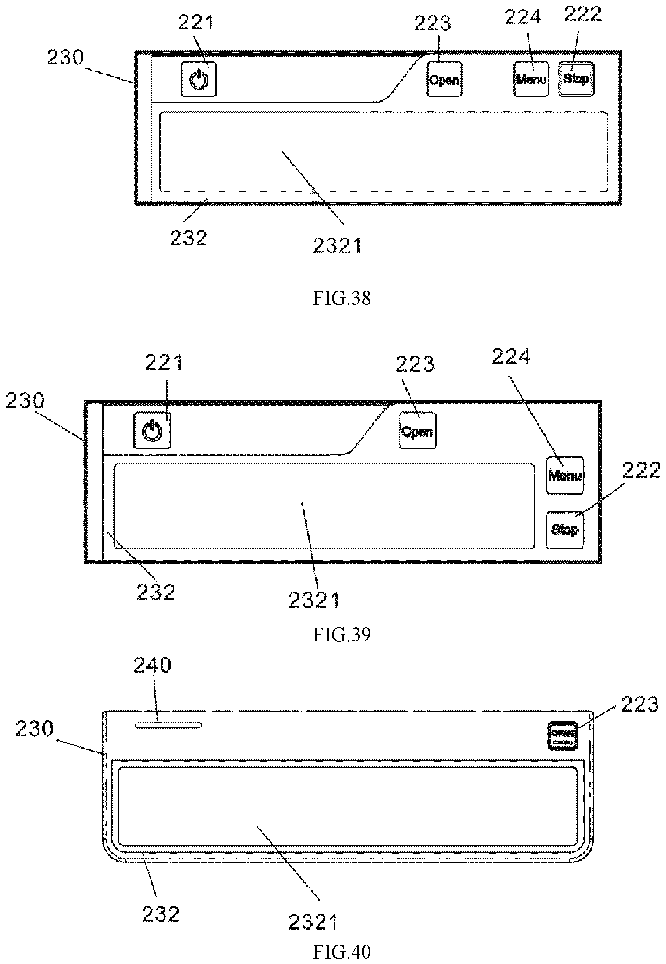

As shown in FIGS. 31-39, in some embodiment, the screen assembly 232 is arranged at a lower portion of the front surface of the pump door 230. The left or right portion of the screen assembly 232 extends upward to enlarge the surface area of the screen assembly 232. When the screen assembly 232 protrudes upward, the protruded portion can extend to the frame 1322 of the door body 231 as shown in FIGS. 34-39 or keep a certain distance from the frame 1322 of the door body 231 as shown in FIGS.31-33.

Of course, in other embodiments, the screen assembly 232 is arranged at an upper portion of the front surface of the pump door 230. The left or right portion of the screen assembly 232 extends downward to enlarge the surface area of the screen assembly 232.

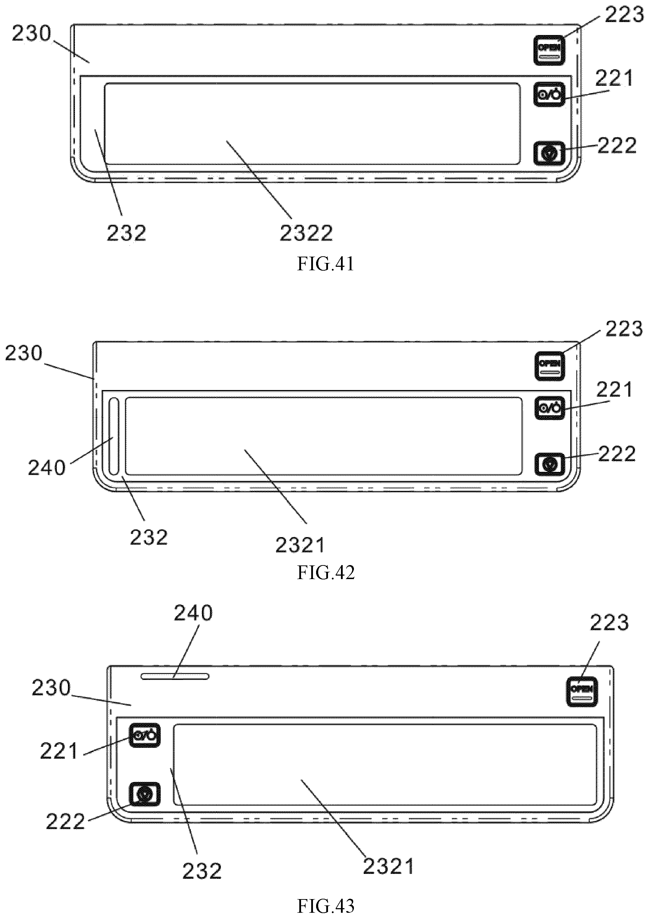

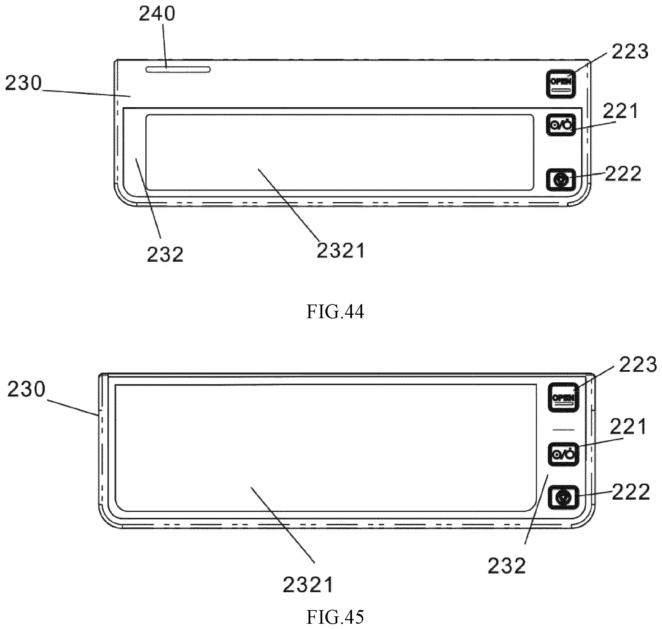

Further, as shown in FIGS. 30-33, 35, 40 and 42-44, in some embodiments, an alarm lamp 240 also included. The alarm lamp 240 is arranged above the screen assembly 232, below the screen assembly 232, on a side of the screen assembly 232, or integrated on the screen assembly 232. In FIGS. 31-33 and 35, the alarm lamp 240 is integrated on the protruded portion which is formed by protruding the left portion of the screen assembly 232. In additional, the alarm lamp 240 can also be integrated on the protruded portion which is formed by protruding the right portion of the screen assembly 232. In FIGS.30, 31, 40, 43-44, the alarm lamp 240 is arranged at the frame 1322 of the door body 231 and at an upper portion of the screen assembly 232. In FIG. 42, the alarm lamp 240 is integrated on the left side of the screen assembly 232. Of course, the alarm lamp 240 can also be arranged at other positions of the pump door 130 and the pump body 210, which will not be illustrated one by one for example herein.

The screen assembly 232 may have a large screen (for example, an integral touch-control display). In addition, please refer to FIGS. 21 and 22. In one embodiment, the screen assembly 232 may also have at least two independent small screens (referring FIGS.21-22). Two adjacent small screens are separated, or two adjacent small screens are separated by an opaque frame. In FIG. 21, the screen assembly 232 has two independent small screens 132a and 132b. In FIG. 22, the screen assembly 232 has three independent small screens 132a, 132b and 132c.

In some embodiments, at least one of these small screens can be selected as an ordinary display screen for displaying information only, and at least one of these small screens can be selected as a touch-control display screen that can display information and realize a touch-control function. Under the combination of these small screens, both display and touch-control functions can be completed. For example, some small screens can be used as the aforementioned real-time display area 1321b, and some small screens can be used as the aforementioned touch-control area 1321a.

In order to further enlarge the surface area of the display area 2321 in the screen assembly 232, in one embodiment, the screen assembly 232 adopts the same structure as the above screen assembly 132. For details, please refer to the structure shown in FIGS. 23 and 24 and the above related description. In this way, the screen assembly 232 with extremely narrow width on the upper and lower sides can be obtained.

In other embodiments, the infusion pump can further be provided with some physical keys 220, which can be an emergency stop key 222, a main menu key 224, and a pump door unlocking key 223, in additional to a power supply key 221. These physical keys 220 may be arranged on the pump door 230 or on the pump body 210.

Referring to FIGS. 28 and 30-45, in some embodiments, the physical key 220 is arranged on the front surface of the pump door 230 and is positioned above the screen assembly 232, below the screen assembly 232, on the side of the screen assembly 232 or integrated on the screen assembly 232. The physical key 220 integrated in the screen assembly 232 may also be arranged at an upper portion, a lower portion or a side of the screen assembly 232.

In one embodiment, some or all of the physical keys 220 are provided on the screen assembly 232. Specifically, the physical key 220 is installed on an outer panel (as shown above) of the screen assembly 232.

As the touch control screen itself is made of glass, it is complicated to add physical keys 220 to the screen. Therefore, generally, the keys of the touch-control display screen use the touch function, and sound, light and vibration are added to improve the perception of the keys for the customer. However, this way cannot achieve the effect of the physical key 220. At the same time, the reliability is not as good as the physical key 220, and the recognizability is not as strong as the physical key 220. However, the physical keys 220 of some non-touch-control display screen are difficult to prevent the liquid or stain residues on the keys. Although the problem of liquid residue can be solved by paster, it is still difficult to achieve the transparent effect of glass in terms of appearance and meanwhile achieve the effect of touch-control display screen.

In this regard, the structure of the physical key 220 shown in an embodiment of the infusion pump adopts the structure of the physical key 120 shown in FIGS 25-27 (the specific structure may refer to the above description). This structure can solve the problem that it is difficult to make the physical key 220 on the glass. At the same time, the hand feel of the physical key 120 can be satisfied while not affecting the flat-faced effect, preventing the liquid inflow and facilitating cleaning. Meanwhile, the requirements of frequent disinfection and cleaning of medical devices can be satisfied.

In this disclosure, specific examples have been used to explain the principle and implementation mode of this disclosure. The description of the above embodiments is only used to help those skilled in the art to understand this disclosure, rather than limit the present disclosure. For those skilled in the technical field of the present disclosure, several simple deductions, deformations or replacements can also be made according to the idea of the present disclosure.

## Claims

1. An injection pump, **characterized in that**, comprising:
a pump body, wherein the pump body is provided with an installation structure which is operable to install a syringe;
a drive device, which is operable to move a piston rod of the syringe to inject a liquid;
a control unit, which is operable to control the drive device;
a physical key, which is electrically connected with the control unit, wherein the physical key is operable for a user to input an instruction, and the physical key comprises a power key; and
a pump door, which is movably installed on the pump body and operable to shield and un-shield the syringe, wherein the syringe is installed on the pump body and the pump door comprises a door body, a transparent window and a screen assembly; wherein the screen assembly is in communication connection with the control unit and has a display area which is capable of displaying information, wherein at least a part of the display area has a touch-control function and the transparent window is installed on the door body; wherein at least an end of the syringe, where a liquid outlet is placed, is capable of being visually shown through the transparent window; wherein a side of the door body, which faces the user, is a front surface of the pump door, the screen assembly is arranged on the front surface of the pump door, and the display area of the screen assembly extends from a left side of a center line of the front surface of the pump door to a right side of the center line of the front surface of the pump door; wherein a width of the display area of the screen assembly is greater than a height of the display area of the screen assembly.

2. The injection pump according to claim 1, **characterized in that**, the width of the display area of the screen assembly is greater than or equal to 70% of a width of the front surface of the pump door.

3. The injection pump according to claim 1 or 2, **characterized in that**, the height of the display area of the screen assembly is greater than or equal to 60% of a height of the front surface of the pump door.

4. The injection pump according to any one of claims 1-3, **characterized in that**, a surface area of the display area of the screen assembly is greater than or equal to 2/3 of a surface area of the front surface of the pump door.

5. The injection pump according to any one of claims 1-4, **characterized in that**, an injection end of the syringe is provided with an extension pipe, and a connection position between the syringe and the extension pipe is visually shown through the transparent window.

6. The injection pump according to any one of claims 1-5, **characterized in that**, a width of the transparent window is greater than or equal to 30% of a width of the front surface of the pump door.

7. The injection pump according to any one of claims 1-6, **characterized in that**, a part of the display area has a touch-control function, information or the instruction inputted by the user through a touch-control mode is displayed on the display area in real time.

8. The injection pump according to any one of claims 1-6, **characterized in that**, an entire display area of the screen assembly is a touch-control display screen with both information display function and touch-control function.

9. The injection pump according to any one of claims 1-8, **characterized in that**:
the transparent window is positioned at an upper portion of the front surface of the pump door, and the screen assembly is positioned below the transparent window; or
the transparent window is positioned at a lower portion of the front surface of the pump door, and the screen assembly is positioned above the transparent window.

10. The injection pump according to claim 9, **characterized in that**:
the transparent window is positioned above the screen assembly, and a left portion or a right portion of the screen assembly extends upward to one side of the transparent window and is arranged in parallel with the transparent window; or
the transparent window is positioned below the screen assembly, and a left portion or a right portion of the screen assembly extends downward to one side of the transparent window and is arranged in parallel with the transparent window.

11. The injection pump according to any one of claims 1-10, **characterized in that**, the physical key is arranged on the front surface of the pump door; wherein the physical key is positioned above the screen assembly, below the screen assembly, or on a side of the screen assembly; or the physical key is integrated on the screen assembly.

12. The injection pump according to any one of claims 1-11, **characterized in that**, the injection pump further comprises an alarm lamp which is positioned above the screen assembly, below the screen assembly, or on a side of the screen assembly, or is integrated on the screen assembly.

13. The injection pump according to any one of claims 1-12, **characterized in that**, the transparent window is made of transparent material, and the transparent window and the screen assembly are jointly sealed with each other.

14. The injection pump according to claim 13, **characterized in that**, the door body has a frame and a support beam which is positioned on the frame, wherein the support beam divides the frame into a transparent window installation area and a screen installation area, wherein the transparent window is fixed inside the transparent window installation area, and the screen assembly is fixed inside the screen installation area.

15. The injection pump according to claim 14, **characterized in that**, the transparent window is made of transparent material, and the transparent window and the frame form an integrated structure through a two-color injection molding process, wherein the screen assembly is fixed and sealed with the support beam and the transparent window by adhesive.

16. The injection pump according to any one of claims 1-15, **characterized in that**, the screen assembly has at least two independent small screens, wherein two adjacent small screens are separated, or two adjacent small screens are separated by an opaque frame.

17. The injection pump according to claim 1, **characterized in that**, the screen assembly comprises an outer panel, a touch-control screen and a display screen; wherein a send terminal and a receive terminal of the display screen are integrated on one display control chip, the touch-control screen is correspondingly provided with a touch-control chip, wherein the display control chip and the touch-control chip are both in communication connection with the control unit; wherein the outer panel has a transparent area and an opaque area which is arranged around the transparent area, wherein the touch-control screen and the display screen are stacked on each other at a back surface of the transparent area, the display area of the display is exposed from the transparent area, and the opaque area has an upper area, a lower area, a first side area and a second side area, wherein the first side area and the second side area are positioned between the upper area and the lower area; wherein the display control chip and the touch-control chip are arranged on a back surface of the first side area, and the second side area is positioned opposite to the first side area.

18. The injection pump according to claim 17, **characterized in that**, a screen test interface is arranged on the back surface of the first side area, and the screen test interface is connected with the display control chip.

19. The injection pump according to claim 17, **characterized in that**, an interface of the display screen and an interface of the touch-control screen are integrated into an integrated interface, wherein the integrated interface is arranged between the first side area and the second side area.

20. The injection pump according to claim 17, **characterized in that**, a respective width of the upper area, the lower area and the second side area of the opaque area is 2-4mm.

21. The injection pump according to claim 17, **characterized in that**, at least some physical keys are arranged inside the second side area.

22. The injection pump according to claim 21, **characterized in that**, a width of the upper area or a width of the lower area is 2-4mm, or a shortest distance from the transparent area of the outer panel to a hole wall of a keyhole of the physical key is 2-4mm.

23. The injection pump according to claim 1, **characterized in that**, the physical key comprises a key switch which is connected with the control unit, a key cap which is sheathed on the key switch, and a support frame which supports the key cap from inside; wherein the screen assembly or the door body is provided with a keyhole, a portion of the key cap protrudes from an inner side of the keyhole and the key cap is matched and sealed with an opening wall of the keyhole.

24. The injection pump according to claim 23, **characterized in that**, the support frame tightly supports an inner wall of the key cap so that the key cap is tightly matched with the opening wall of the keyhole to realize a sealing connection.

25. The injection pump according to any one of claims 1-24, **characterized in that**, the installation structure is positioned inside the pump door and is operable to fix the syringe.

26. An injection pump, **characterized in that**, comprising:
a pump body, wherein the pump body is provided with an installation structure which is operable to install a syringe;
a drive device, which is operable to move a piston rod of the syringe to inject a liquid;
a control unit, which is operable to control the drive device;
a physical key, which is electrically connected with the control unit, wherein the physical key is operable for a user to input an instruction, and the physical key comprises a power key; and
a pump door, which is movably installed on the pump body and operable to shield and un-shield an infusion pipe which is installed on the pump body, wherein the pump door comprises a door body, a transparent window and a screen assembly; wherein the screen assembly is in communication connection with the control unit and has a display area which is capable of displaying information, wherein at least a part of the display area has a touch-control function and the transparent window is installed on the door body; wherein at least an end of the syringe, where a liquid outlet is placed, is capable of visually shown through the transparent window, wherein the syringe is installed on the pump body; wherein a side of the door body, which faces the user, is a front surface of the pump door, the screen assembly is arranged on the front surface of the pump door, wherein a width of the display area of the screen assembly is greater than or equal to 70% of a width of the front surface of the pump door, and a width of the transparent window is greater than or equal to 30% of the width of the front surface of the pump door.

27. The injection pump according to claim 26, **characterized in that**, a height of the display area of the screen assembly is greater than or equal to 60% of a height of the front surface of the pump door.

28. The injection pump according to claim 26 or 27, **characterized in that**, a surface area of the display area of the screen assembly is greater than or equal to 2/3 of a surface area of the front surface of the pump door.

29. An infusion pump, **characterized in that**, comprising:
a pump body, wherein the pump body is provided with a liquid stopping structure which is operable to stop a liquid which flows through an infusion pipe;
a drive device, which is operable to drive the liquid to flow into the infusion pipe;
a control unit, which is operable to control the drive device;
a physical key, which is electrically connected with the control unit, wherein the physical key is operable for a user to input an instruction, and the physical key comprises a power key; and
a pump door, which is movably installed on the pump body and operable to shield and un-shield the infusion pipe, wherein the infusion pipe is installed on the pump body, and the pump door comprises a door body and a screen assembly which is capable of displaying information, wherein the screen assembly is in communication connection with the control unit and has a display area which is capable of displaying information, wherein at least a part of the display area has a touch-control function; wherein a side of the door body, which faces the user, is a front surface of the pump door, the screen assembly is arranged on the front surface of the pump door, and the display area of the screen assembly extends from a left side of a center line of the front surface of the pump door to a right side of the center line of the front surface of the pump door, wherein a width of the display area of the screen assembly is greater than a height of the display area of the screen assembly.

30. The infusion pump according to claim 29, **characterized in that**, the width of the display area of the screen assembly is greater than or equal to 70% of a width of the front surface of the pump door.

31. The infusion pump according to claim 29 or 30, **characterized in that**, the height of the display area of the screen assembly is greater than or equal to 60% of a height of the front surface of the pump door.

32. The infusion pump according to any one of claims 29-31, **characterized in that**, a surface area of the display area of the screen assembly is greater than or equal to 2/3 of a surface area of the front surface of the pump door.

33. The infusion pump according to any one of claims 29-32, **characterized in that**, a part of the display area has a touch-control function, information or the instruction inputted by the user through a touch-control mode is displayed on the display area in real time.

34. The infusion pump according to any one of claims 29-33, **characterized in that**, an entire display area of the screen assembly is a touch-control display screen with both information display function and touch-control function.

35. The infusion pump according to any one of claims 29-34, **characterized in that**:
the screen assembly is positioned at a lower portion of the front surface of the pump door; or
the screen assembly is positioned at an upper portion of the front surface of the pump door.

36. The infusion pump according to any one of claims 29-35, **characterized in that**:
the screen assembly is positioned at a lower portion of the front surface of the pump door, and a left portion or a right portion of the screen assembly extends upward to an upper portion of the front surface of the pump door for enlarging a surface area of the screen assembly; or
the screen assembly is positioned at an upper portion of the front surface of the pump door, and a left portion or a right portion of the screen assembly extends downward to a lower portion of the front surface of the pump door for enlarging a surface area of the screen assembly.

37. The infusion pump according to any one of claims 39-36, **characterized in that**, the physical key is arranged on the front surface of the pump door; wherein the physical key is positioned above the screen assembly, below the screen assembly, or on a side of the screen assembly; or the physical key is integrated on the screen assembly.

38. The infusion pump according to any one of claims 29-37, **characterized in that**, the infusion pump further comprises an alarm lamp which is positioned above the screen assembly, below the screen assembly, or on a side of the screen assembly, or is integrated on the screen assembly.

39. The infusion pump according to any one of claims 29-38, **characterized in that**, the screen assembly has at least two independent small screens, wherein two adjacent small screens are separated, or two adjacent small screens are separated by an opaque frame.

40. The infusion pump according to claim 29, **characterized in that**, the screen assembly comprises an outer panel, a touch-control screen and a display screen; wherein a send terminal and a receive terminal of the display screen are integrated on one display control chip, the touch-control screen is correspondingly provided with a touch-control chip, wherein the display control chip and the touch-control chip are both in communication connection with the control unit; wherein the outer panel has a transparent area and an opaque area which is arranged around the transparent area, wherein the touch-control screen and the display screen are stacked on each other at a back surface of the transparent area, the display area of the display is exposed from the transparent area, and the opaque area has an upper area, a lower area, a first side area and a second side area, wherein the first side area and the second side area are positioned between the upper area and the lower area; wherein the display control chip and the touch-control chip are arranged on a back surface of the first side area, and the second side area is positioned opposite to the first side area.

41. The infusion pump according to claim 40, **characterized in that**, a screen test interface is arranged on the back surface of the first side area, and the screen test interface is connected with the display control chip.

42. The infusion pump according to claim 40, **characterized in that**, an interface of the display screen and an interface of the touch-control screen are integrated into an integrated interface, wherein the integrated interface is arranged from the first side area toward a center of the display screen along a length direction of the display screen.

43. The infusion pump according to claim 40, **characterized in that**, a respective width of the upper area, the lower area and the second side area of the opaque area is 2-4mm.

44. The infusion pump according to claim 40, **characterized in that**, at least some physical keys are arranged inside the second side area.

45. The infusion pump according to claim 44, **characterized in that**, a width of the upper area or a width of the lower area is 2-4mm, or a shortest distance from the transparent area of the outer panel to a hole wall of a keyhole of the physical key is 2-4mm.

46. The infusion pump according to claim 29, **characterized in that**, the physical key comprises a key switch which is connected with the control unit, a key cap which is sheathed on the key switch, and a support frame which supports the key cap from inside; wherein the screen assembly or the door body is provided with a keyhole, a portion of the key cap protrudes from an inner side of the keyhole and the key cap is matched and sealed with an opening wall of the keyhole.

47. The infusion pump according to claim 43, **characterized in that**, the support frame tightly supports an inner wall of the key cap so that the key cap is tightly matched with the opening wall of the keyhole to realize a sealing connection.

48. An infusion pump, **characterized in that**, comprising:
a pump body, wherein the pump body is provided with a liquid stopping structure which is operable to stop a liquid which flows through an infusion pipe;
a drive device, which is operable to drive the liquid to flow into the infusion pipe;
a control unit, which is operable to control the drive device;
a physical key, which is electrically connected with the control unit, wherein the physical key is operable for a user to input an instruction, and the physical key comprises a power key; and
a pump door, which is movably installed on the pump body and operable to shield and un-shield a syringe, wherein the syringe is installed on the pump body, and the pump door comprises a door body and a screen assembly which is capable of displaying information, wherein the screen assembly is in communication connection with the control unit and has a display area which is capable of displaying information, wherein at least a part of the display area has a touch-control function; wherein a side of the door body, which faces the user, is a front surface of the pump door, and the screen assembly is arranged on the front surface of the pump door, wherein a width of the display area of the screen assembly is greater than or equal to 70% of a width of the front surface of the pump door.

49. The infusion pump according to claim 48, **characterized in that**, a height of the display area of the screen assembly is greater than or equal to 60% of a height of the front surface of the pump door.

50. The infusion pump according to claim 48 or 49, **characterized in that**, a surface area of the display area of the screen assembly is greater than or equal to 2/3 of a surface area of the front surface of the pump door.
